# EUROPÄISCHE PATENTSCHRIFT

(11) **EP 0 494 898 B1**
(45) Veröffentlichungstag und Bekanntmachung des Hinweises auf die Patenterteilung: **15.02.1995**
(21) Anmeldenummer: 90914392.7
(22) Anmeldetag: 25.09.1990
(51) Int. Cl.: B01J 23/86, C07C 29/149

(54) **VERFAHREN ZUR HERSTELLUNG VON SÄUREFESTEN KATALYSATOREN FÜR DIE DIREKTE HYDRIERUNG VON CARBONSÄUREN ZU ALKOHOLEN**
PROCESS FOR PRODUCING ACID-RESISTANT CATALYSTS FOR THE DIRECT HYDRATION OF CARBOXYLIC ACIDS INTO ALCOHOLS
PROCEDE DE FABRICATION DE CATALYSEURS RESISTANT AUX ACIDES POUR L'HYDROGENATION DIRECTE DES ACIDES CARBOXYLIQUES EN ALCOOLS

(30) Priorität: 04.10.1989 DE 3933138; 20.12.1989 DE 3942064
(43) Veröffentlichungstag der Anmeldung: 22.07.1992
(73) Patentinhaber: Henkel Kommanditgesellschaft auf Aktien, 40191 Düsseldorf (DE)
(72) Erfinder: POHL, Joachim, Dr., D-4000 Düsseldorf (DE); CARDUCK, Franz-Josef, Dr., D-5657 Haan (DE); GÖBEL, Gerd, Dr., D-5000 Köln 40 (DE)
(86) Internationale Anmeldenummer: EP9001624
(87) Internationale Veröffentlichungsnummer: WO9104789

(56) Entgegenhaltungen:
- EP-A- 0 280 982
- EP-A- 0 300 347
- DE-A- 2 250 844
- DE-A- 2 611 374
- FR-A- 889 791
- FR-A- 2 439 176
- US-A- 3 899 577

## Beschreibung

### Gebiet der Erfindung

Die Erfindung betrifft ein Verfahren zur Herstellung von säurefesten Cupfer-Chrom-Katalysatoren sowie die Verwendung der Katalysatoren für die Hydrierung von Carbonsäuren zu Alkoholen.

### Stand der Technik

Die Hydrierung von Carbonsäuren, technisch interessant ist vor allen Dingen die Hydrierung freier Fettsäuren zu Fettalkoholen, kann nach **Ullmanns Encyklopädie der technischen Chemie, IV. Auflage, Band 11, Seiten 427 - 445,** in Gegenwart von Cupferchromit-Katalysatoren durchgeführt werden. Nach einem von Adkins erarbeiteten Verfahren [**Zt.Anorg.Allgm. Chem., 346, 66 (1966**]) werden gewöhnlich Cupferchromit-Katalysatoren über intermediär gebildetes Cupferammoniumhydroxychromat, das durch Calzinierung, d.h. thermisch, in Cupferoxid und Cupferchromit zersetzt wird, gewonnen. Das intermediär gebildete Cupferammoniumhydroxychromat erhält man durch Niederschlagsbildung von Ammoniumchromat und Cupfer(II)salzen.

Diese Cupferchromit-Katalysatoren lassen sich jedoch nur dann brauchbar einsetzen, wenn das durch Zersetzung des primär gebildeten Cupferammoniumhydroxychromat entstehende, säurelösliche Cupfer(II)oxid anschließend durch Säurebehandlung, z.B. mit Eisessig, herausgewaschen wird. Derartig säuregewaschene Kupferchromit-Katalysatoren sind jedoch teuer, unzureichend tablettierbar und dementsprechend nicht in abriebfeste und mechanisch stabile Strangpreßlinge oder andere Formkörper zu überführen. Daher sind jene säuregewaschenen Katalysatoren nur für die Suspensionshydrierung von Carbonsäuren brauchbar. Diese Verfahren erfordern jedoch im Vergleich zu festbettkatalytischen Prozessem zum einen verhältnismäßig große Reaktoren und erbringen zum anderen schlechtere Umsatzraten.

In der deutschen Offenlegungsschrift **DE-A1 37 06 658** wird ein Verfahren zur Herstellung eines säurefesten Cupferchromit-Spinell-Katalysators für die direkte Festbetthydrierung von Fettsäuren beschrieben. Dazu wird Cupfer(II)chromit, hergestellt über intermediär gebildetes Cupferammoniumhydroxychromat, in Gegenwart von kolloidalem Kieselgel in an sich bekannter Weise dargestellt, und bei Temperaturen um 750°C im Verlauf von wenigstens 12 Stunden geglüht. Der nach obiger Offenlegungsschrift hergestellte Cupfer(II)chromit-Spinell-Katalysator enthält als Promotoren, die vermutlich durch Anhebung elektronischer Zustände im Katalysator die Hydrierung erleichtern, Barium- und Mangansalze in Mengen von jeweils unter 5 Gew.-% - berechnet als Metall und bezogen auf Feststoffbestandteile der Katalysatorbildenden Reaktionsmischung.

Aus der europäischen Patentanmeldung **EP-A1 0 280 982** sind des weiteren oxidische Hydrierkatalysatoren mit einem Gehalt an Chrom, Barium, Cupfer, Mangan und Silicium bekannt, die jedoch unter milden Bedingungen bis maximal 750°C calciniert werden und keine zufriedenstellende Säurebeständigkeit aufweisen.

Eine Verbesserung der Katalysatoraktivität von Cupferchromit-Katalysatoren durch Zusatz von Mangan und/oder Bariumsalzen in Mengen bis zu 10 Gew.-% ist ebenfalls aus den europäischen Patentschriften **EP-B 0 069 339, EP-B 0 276 722** und **EP-B 0 023 699** bekannt.

Zur Bestimmung der Katalysatoraktivität eignet sich die sogenannte Abschreibung eines Katalysators. Unter der Abschreibung eines Katalysators versteht man den Quotienten aus Gewicht des Katalysators und der Menge an eingesetzter Fettsäure während der Betriebszeit, der vorteilhaft in Prozenten ausgedrückt wird. Die Abschreibung ist somit indirekt ein Maß für die Katalysatoraktivität und Standzeit, da die Menge an eingesetzter Fettsäure dem Umsatz an Fettalkohol proportional ist. Katalysatoren, die eine Abschreibung unter 0,3 % erreichen, werden gemeinhin als gut bezeichnet.

Die nach der deutschen Offenlegungsschrift **DE-A1 37 06 658** hergestellten Cupfer(II)chromit-Spinell-Katalysatoren konnten zwar eine Abschreibung von 0,28 erreichen, sind aber trotz allem noch zu verbessern.

Aufgabe der vorliegenden Erfindung ist daher die Herstellung von Katalysatormassen, die für die direkte Festbetthydrierung von Carbonsäuren geeignet sind, und die sich durch Säurebeständigkeit sowie eine guten Abschreibung im oben genannten Sinne auszeichnen.

### Beschreibung der Erfindung

Gegenstand der vorliegenden Erfindung ist somit ein Verfahren zur Herstellung von säurefesten, zur direkten Hydrierung von Carbonsäuren zu Alkoholen geeigneten Cupfer-Chrom-Katalysatoren, das sich dadurch auszeichnet, daß man
a) wäßrige Lösungen, enthaltend (a1) Chrom(VI)oxid und (a2) alkalisch stabilisierte kolloidale Kieselsäure in Mengen von 2 bis 15 Gew.-% - berechnet als Feststoff an Siliciumdioxid und bezogen auf den Feststoffgehalt der Lösung - mit im wäßrigen Milieu alkalisch reagierenden Verbindungen versetzt,
b) die gebildeten Suspensionen mit wäßrigen Lösungen, enthaltend Salze des (b1) Cupfers, (b2) Bariums in Mengen von 0,2 bis 0,9 Gew.-% - berechnet als Metall und bezogen auf den fertigen Katalysator - und (b3) Mangans in Mengen von 3 bis 6 Gew.-% - berechnet als Metall und bezogen auf den fertigen Katalysator - sowie weitere Salze von Metallen ausgewählt aus der Gruppe, die von Calcium, Magnesium, Aluminium, Nickel, Eisen, Cer, Lanthan und Silber gebildet wird, in Mengen von 30 bis 100 Mol-% - berechnet als Metall und bezogen auf den Cupfer(II)oxid-Anteil im fertigen Katalysator - behandelt,
c) die Reaktionsmischungen bei erhöhter Temperatur zusammenmischt sowie
d) die gebildeten Feststoffe abtrennt, trocknet, bei einer Temperatur von 750 bis 1050°C calciniert, gegebenenfalls granuliert und anschließend in stückige Form bringt.

Überraschenderweise konnten derart verbesserte Kupfer-Chromit-Katalysatoren dadurch hergestellt werden, daß wenigstens der überwiegende Anteil des säurelöslichen Cupfer(II)oxids, das nach der thermischen Zersetzung des intermediär gebildeten Cupferammoniumhydroxychromats im Gemisch mit Cupferchromit vorliegt, mit Fremdmetallverbindungen in Gegenwart von kolloidaler Kieselsäure zu säurefesten Reaktionsfolgeprodukten abreagiert.

### Herstellung der Katalysatoren

Die für das erfindungsgemäße Verfahren eingesetzten Cupferchromit und Cupfer(II)oxid, die kolloidale Kieselsäure sowie Fremdmetallverbindungen enthaltenden Feststoffmischungen, die einer Hochtemperatur-Festkörperreaktion unterworfen werden, können erfindungsgemäß nach zwei verschiedenen Verfahren hergestellt werden:
a) Vermahlen und intensive Vermischung der als Feststoffe eingesetzten Komponenten
b) Fällung der entsprechenden löslichen Metallverbindungen in Gegenwart von kolloidaler Kieselsäure und anschließende thermische Zersetzung.

Im Rahmen der Erfindung wird die Herstellung der Cupferchromit, Cupferoxid, Fremdmetallverbindungen sowie kolloidale Kieselsäure enthaltenden Feststoffmischung in Anlehnung an den Weg b) bevorzugt. Danach werden zunächst die Fremdmetallverbindungen sowie die Cupfer- und Chromverbindung in ihre wäßrigen Lösung überführt und in Gegenwart von kolloidaler Kieselsäure durch Copräzipitation gefällt.

Nach einer besonders bevorzugten Ausführungsform wird die Chrom(VI)verbindung in Gegenwart von Ammoniak in ihre wäßrige Lösung überführt und mit kolloidalem Kieselsäuresol versetzt. In einem separaten Behälter werden die Cupfer- sowie Fremdmetallverbindungen in ihre wäßrigen Lösungen überführt. Die Copräzipitation erfolgt erfindungsgemäß durch Zugabe der Lösung des einen Behälters zu der des anderen Behälters. Gemäß einer besonderen Ausführungsform der vorliegenden Erfindung erfolgt die Copräzipitation durch Pumpen der einen Lösung zur anderen, wodurch man eine besonders homogene Verteilung der unlöslichen Verbindungen erhält. Im Sinne der Erfindung kann die Copräzipitation auch durchaus in Gegenwart anderer, im wäßrigen Milieu alkalisch reagierender Verbindungen wie beispielsweise in Gegenwart von Alkali-, Erdalkalihydroxiden und/oder alkalisch reagierender Amine durchgeführt werden. Erfindungsgemäß bevorzugt ist die ammonikalische Fällung, da der danach erhaltene Niederschlag verhältnismäßig leicht thermisch zersetzt werden kann. Der aus der ammoniakalischen Fällung gewonnene Niederschlag, der die schwerlöslichen Fremdmetallverbindungen, das Cupferammoniumhydroxychromat sowie die kolloidale Kieselsäure enthält, wird, gegebenenfalls nach Auswaschen störender Anionen, thermisch zersetzt. Dabei entsteht aus dem intermediär gebildeten Cupferammoniumhydroxychromat Cupferchromit sowie säurelösliches Cupfer(II)oxid, die in inniger Vermischung neben den schwerlöslichen Fremdmetallverbindungen sowie der kolloidalen Kieselsäure vorliegen.

### Fremdmetallverbindungen

In dem erfindungsgemäßen Verfahren werden als Fremdmetallverbindungen Salze von Calcium, Magnesium, Aluminium, Nickel, Eisen, Cer, Lanthan und/oder Silber zugesetzt. Als reaktive Zusatzstoffe sind aus dieser Gruppe solche Fremdmetalle bevorzugt, die mit dem säurelabilen Cupferoxid unter den Bedingungen der Hochtemperatur-Festkörperreaktion säurefeste multiple Oxidverbindungen, vorzugsweise vom Spinell-Typ bilden. Erfindungsgemäß werden diese reaktiven Fremdmetalle in Mengen von 30 bis zu 100 mol%, vorzugsweise von mindestens 50 mol-% und insbesondere mindestens 75 mol-% - berechnet als Fremdmetall und bezogen auf jeweils im Katalysator-Vorprodukt vorliegendes oder sich intermediär bildendes Cupfer(II)oxid - zugesetzt. Die Menge der zugesetzten Fremdmetallverbindungen wird nach obenhin so begrenzt, daß die Bildung substantieller Mengen säurelabiler Fremdmetallfolgeprodukte ausgeschlossen wird. Im Rahmen der Erfindung hat sich als besonders günstige Ausführungsform der Zusatz von Manganverbindungen in wenigstens äquimolarer Menge zu vorliegendem oder sich intermediär bildendem Cupferoxid erwiesen. Vorzugsweise liegt das Mangan/Cupfer-Molverhältnis in einem Bereich von 1,8 bis 2 - bezogen auf säurelabiles Cupferoxid,
In einer besonderen Ausführungsform der vorliegenden Erfindung werden unabhängig von der Menge der eingesetzten reaktiven Fremdmetallzusätze zusätzlich Barium- und Mangansalze als Promotoren vor der Copräzipitation zugesetzt. Ein Zusatz an Bariumsalzen in Mengen von unter 2 Gew.-%, vorzugsweise von 0,2 bis 0,9 Gew.-% - berechnet als Metall und bezogen auf fertigen Katalysator - steigert die Aktivität des Katalysators nach der Hochtemperaturreaktion besonders. Ein Zusatz an Mangansalzen in Mengen von unter 6 Gew.-%, vorzugsweise unter 5 Gew.-% und über 3 Gew.-% - berechnet als Metall und bezogen auf fertigen Katalysator - steigert vergleichbar mit dem Zusatz an Bariumsalzen die Aktivität des Katalysators. Auch diese Menge an zugesetzten Mangansalzen wird unabhängig von der Menge an reaktiven Fremdmetalle berechnet. Da bekannt ist, daß Barium- und Mangansalzen in jenen geringen Mengen als Promotoren wirken, werden sie in ihrer Wirkung und daher auch mengenmäßig unabhängig von der der reaktiven Fremdmetalle als Bildner von säurefesten multiplen Oxiden betrachtet.

### Kolloidale Kieselsäure

Im Rahmen der Erfindung hat es sich als besonders vorteilhaft erwiesen, alkalisch stabilisierte, kolloidale Kieselsäure vor der Copräzipitation zuzusetzten. Es wurde gefunden, daß die kolloidale alkalisch stabilisierte Kieselsäure in Mengen von 2 bis 15 Gew.-%, vorzugsweise in Mengen von 3 bis 10 Gew.-% - berechnet als Feststoff an Siliciumdioxid und bezogen auf Feststoffmischung bildendes Einsatzgemisch - die Porosität des Spinells unter Hochtemperaturbedingungen aufrecht erhält. Eine Menge an kolloidaler Kieselsäure über 15 Gew.-% ist zu vermeiden, da damit die Gefahr einer Zusammenballung der Kieselsäure bzw. die allgemein bekannte, für das erfindungsmäßige Verfahren jedoch unerwünschte, Bindemitteleigenschaft der Kieselsäure wächst.

Im Sinne der Erfindung können die im Handel erhältlichen, kolloidalen, alkalisch stabilisierten Kieselsäuresole eingesetzt werden. Die Herstellung und die Eigenschaften jener Kieselsäuresole werden beispielsweise in den amerikanischen Patentschriften **US 2 574 902** und **US 3 146 210** beschrieben. Bei dem erfindungsgemäßen Verfahren werden Kieselsäuresole eingesetzt, deren meist kugelförmige Siliciumdioxid-Partikel einen Durchmesser von höchstens 30 nm haben. Als Stabilisierungsmittel für die Siliciumdioxidpartikel werden im wäßrigen Milieu lösliche, alkalisch reagierende Verbindungen bevorzugt. Insbesondere der leichtflüchtige Ammoniak ist ein geeignetes Stabilisierungsmittel, um den Solcharakter der Siliciumdioxid-Partikel zu erhalten.

### Calcinierung

Erfindungsgemäß werden die Cupferchromit, Cupfer(II)oxid und kolloidale Kieselsäure sowie Fremdmetallverbindungen enthaltenden Feststoffmischungen einer Hochtemperatur-Festkörperreaktion unterworfen, wobei wenigstens der überwiegende Anteil des Cupferoxids mit den reaktiven Fremdmetallverbindungen zu säurefesten Reaktionsfolgeprodukten abreagiert.

Erfindungsgemäß kann die Hochtemperatur-Festkörperreaktion derartiger Feststoffmischungen entweder satzweise im Kammerofen oder kontinuierlich im Drehofen bei Temperaturen von 750 bis 1050 und insbesondere zwischen 850 und 950°C durchgeführt werden.

Erfindungsgemäß wird die satzweise Festkörperreaktion bei Temperaturen des angegebenen Bereichs, vorzugsweise bis 950°C und bei einer Reaktionsdauer bis zu 24 Stunden, vorzugsweise bis etwa 12 Stunden durchgeführt. Die kontinuierliche Festkörperreaktion wird erfindungsgemäß bei den Temperaturen des angegebenen Bereichs, bevorzugt oberhalb 850°C und mit einer Reaktionsdauer deutlich unter einer Stunde durchgeführt. Einer Ausführungsform der vorliegenden Erfindung entsprechend kann die Festkörperreaktion der Feststoffmischungen, die Mangan-Verbindungen als reaktive Fremdmetallzusätze enthalten, satzweise im Kammerofen bevorzugt bei 750°C mit einer Reaktionsdauer von vorzugsweise 12 Stunden oder kontinuierlich im Drehofen bevorzugt bei 900°C mit einer Reaktionsdauer von vorzugsweise unter einer Stunde durchgeführt werden.

Festkörperreaktionen von Feststoffmischungen mit Aluminium- und/oder Nickel- und/oder Eisen-Verbindungen als reaktive Fremdmetallzusätze können ebenfalls satzweise oder kontinuierlich durchgeführt werden, wobei mit höheren Reaktionstemperaturen, bevorzugt oberhalb von 850°C, gearbeitet wird.

Entsprechend einer besonderen Ausführungsform der vorliegenden Erfindung erfolgt die thermische Zersetzung des Copräzipitats in die Cupferchromit, Cupfer(II)oxid und kolloidale Kieselsäure sowie Fremdmetallverbindungen enthaltende Feststoffmischung sowie die Hochtemperatur-Festkörperreaktion zu den säurefesten Reaktionsfolgeprodukten in einem Durchgang, d.h. ohne Isolierung der nach der thermischen Zersetzung vorliegenden Feststoffmischung. Es wird bevorzugt, den getrockneten Niederschlag (Copräzipitat) in den jeweiligen Ofen zu geben und diesen bis zur gewünschten Calcinierungstemperatur aufzuheizen. Während dieser Aufheizphase, vermutlich über 400°C, zersetzt sich das Copräzipitat thermisch in die Feststoffmischung, die dann bei den oben beschriebenen Calcinierungstemperaturen der Hochtemperatur-Festkörperreaktion unterworfen wird.

### Formgebung

Die nach dem erfindungsgemäßen Verfahren hergestellten Katalysatormassen fallen als Pulver an und sind äußerst säureresistent sowie katalytisch aktiv. Sie weisen spezifische Oberflächen nach BET im Bereich von 4 bis 50 m²/g, vorzugsweise von etwa 8 bis 20 m²/g auf. Desweiteren zeigen die nach dem erfindungsgemäß hergestellten katalytisch aktiven Massen ein primäres Korngrößenmaximum im Bereich von etwa 3 bis 60 »m.

Die erfindungsgemäß hergestellten pulverförmigen Katalysatormassen können formgebend weiterverarbeitet werden unter Zusatz üblicher Binde- und/oder Gleitmittel, vorzugsweise in Mengen nicht über 10 Gew.-% - bezogen auf nach der Hochtemperatur-Festkörperreaktion erhaltene Katalysatormasse -, wobei als Bindemittel bevorzugt Polyvinylacetat, Polyvinylalkohol und/oder Methylcellulose und als Gleitmittel insbesondere Graphit eingesetzt werden.

Besonders bevorzugt ist die Weiterverarbeitung der katalytisch aktiven Massen zu Katalysatorformlingen, wobei insbesondere die physikalischen Daten dieser Formlinge in den folgenden Bereichen liegen:
*** Bruchhärte 3 bis 10 kp pro Formling und
*** Porenvolumen 0,1 bis 0,6 cm³/g, vorzugsweise 0,2 bis 0,4 cm³/g.

### Katalysatormassen

In den hergestellten säurefesten Katalysatormassen liegen Umsetzungsprodukte des Cupferoxids beispielsweise mit Nickel, Eisen, Aluminium und/oder vorzugsweise Manganverbindungen in Form säurefester multipler Oxide, insbesondere in Form von Spinell-Verbindungen vor. Die nach dem erfindungsgemäßen Verfahren erhältlichen säurefesten Katalysatormassen enthalten in Form säurefester multipler Oxide die Fremdmetalle in Mengen von etwa 2 bis 30 Gew.-%, vorzugsweise in Mengen von etwa 10 bis 18 Gew.-% - jeweils bezogen auf Gesamtkatalysator. Desweiteren enthalten die erfindungsgemäßen säurefesten Katalysatormassen Barium- und Mangansalze als Promotoren, die nicht unter die reaktionsfähigen Fremdmetallverbindungen gezählt werden. Bariumsalze sind in Mengen von unter 2 Gew.-%, vorzugsweise von 0,2 bis 0,9 Gew.-% und Mangansalze sind in Mengen von unter 6 Gew.-% , vorzugsweise von 3 bis 5 Gew.-% - berechnet als Metall und bezogen auf fertigen Katalysator - enthalten. Als weitere wichtige Komponente enthalten die erfindungsgemäßen säurefesten Katalysatormassen homogen eingearbeitet kolloidale Kieselsäure, in Mengen von 2 bis 15 Gew.-%, vorzugsweise in Mengen von 3 bis 10 Gew.-% - berechnet als Feststoff und bezogen auf Katalysatorbildendes Vorprodukt.

Die nach dem erfindungsgemäßen Verfahren erhältlichen Katalysatormassen zeigen eine verbesserte Abschreibung im oben genannten Sinne mit Werten unter 0,28 %, vorzugsweise von 0,2 bis 0,25 %.

### Gewerbliche Anwendbarkeit

Ein weiterer Gegenstand der Erfindung betrifft die Verwendung der säurefesten Katalysatormassen, die nach dem obigen Verfahren hergestellt worden sind, zur direkten Hydrierung von Carbonsäuren zu Alkoholen, vorzugsweise von Fettsäuren zu Fettalkoholen nach dem Verfahrenstyp der Festbetthydrierung. Erfindungsgemäß werden die säurefesten Katalysatoren üblicherweise vor ihrem Einsatz in der Direkthydrierung von Fettsäuren mit Wasserstoff oder einem Wasserstoff-enthaltenden Gasgemisch aktiviert. Mit Vorteil wird zur Aktivierung der Katalysatormassen ein Gasgemisch verwendet, das überwiegend aus einer Stickstoff/Wasserstoff-Gasmischung besteht.

Vorteilhafterweise kann eine derartige Aktivierung, wie aus dem Stand der Technik bekannt, so durchgeführt werden, daß die Katalysatormassen nach der Herstellung im Stickstoffstrom bei erhöhter Temperatur getrocknet werden und im trocknenden Gas in steigenden Mengen Wasserstoff zur Aktivierung beigemischt wird. Dabei kann der Wasserstoffanteil in dem aktivierenden Gasgemisch im Bereich von 0,1 bis 10 Volumen-% liegen. Die Aktivierung der Katalysatoren kann dabei sowohl in situ als auch in von dem Reaktionsgefäß getrennten Gefäßen durchgeführt werden.

Im Rahmen der Erfindung werden die Katalysatormassen vorzugsweise bei der Hydrierung von Fettsäuren und/oder Fettsäuregemische, insbesondere solche natürlichen Ursprungs mit 6 bis 24 C-Atomen, die auch ein und/oder mehrfach olefinisch ungesättigt sein können, verwendet.

Erfindungsgemäß werden die Katalysatormassen in einem Direkthydrierverfahren verwendet, in dem man mit einem Volumenverhältnis von Katalysatormenge zu Fettsäuregemisch im Bereich von 0,1 bis 3 pro Stunde arbeitet. Die im Einzelfall einzustellende Reaktionsparameter werden u.a. durch die Länge der Kohlenstoffketten der zu reduzierenden Fettsäuren oder Fettsäuregemische mitbestimmt. Je kürzer die Kettenlänge der eingesetzten Fettsäuren ist, umso niedriger sind im allgemeinen die Reaktionstemperaturen, die innerhalb der Bereiche des allgemeinen Standes der Technik liegen. Bevorzugt wird bei Reaktionstemperaturen zwischen 220 bis 280°C gearbeitet. Die Verfahrensdrucke liegen im allgemeinen bei 200 bar und darüber, insbesondere im Bereich von 200 bis 500 bar. Prinzipiell führt die Verwendung von höheren Drucken zur Senkung der Säurezahl der Reaktionsprodukte und damit zu einer Steigerung der Ausbeute an gewünschten Fettalkoholen.

Die folgenden Beispiele erläutern die Herstellung und den Einsatz der säurefesten Hydrierkatalysatoren, ohne ihn darauf zu beschränken.

### Beispiele

Der Katalysator wurde nach einem gleichbleibendem Verfahren hergestellt, das vorab ausführlich beschrieben wird. In den Beispielen 1 bis 12 werden die Mengen der einzelnen Komponenten angegeben. Die Calcinierung der Katalysatoren und der Einsatz als Hydrierkatalysator im Rührautoklaven werden exemplarisch in Beispiel 13 beschrieben, die konkreten Angaben zu den einzelnen Katalysatoren sind in Tabelle 1 zusammengefaßt. Im Beispiel 14 wird der Einsatz eines ausgewählten Hydrierkatalysators für die Fettsäurehydrierung im Hochdruckrohrreaktor beschrieben.

### I. Herstellung der Katalysatoren

In dem Rührbehälter 1 wurde Chrom(VI)oxid in entionisiertem Wasser gelöst und mit 25%igem Ammoniak in eine ammoniakalische Ammoniumhydroxychromat-Lösung überführt. Diese wurde unter Rühren mit einem alkalisch stabilisierten Kieselsäuresol versetzt und auf 70°C erhitzt. In einem zweiten Rührbehälter wurden die Nitrate von Barium, Mangan, Kupfer und die der Fremdmetalle in entionisiertem Wasser gelöst und ebenfalls auf 70°C erhitzt. Die Niederschlagsbildung erfolgte durch Pumpen der Metallionen-Lösung des Behälters 2 in die AmmoniumhydroxychromatLösung des Behälters 1 und war bei einem pH-Wert von 6,3 beendet. Der Niederschlag wurde abfiltriert, nitratfrei gewaschen und bei etwa 100°C getrocknet.

### Beispiel 1: (Cr/Cu/Si/Ba/Mn/La)

In Rührbehälter 1 wurden 187,5 g Chromoxid, gelöst in 1,2 l Wasser, mit 1,8 l 25%igem Ammoniak und 62,8 g Ludox AS 40 unter Rühren versetzt. In Rührbehälter 2 wurden 11,0 g Bariumnitrat, 38,2 g Mangannitrat · 4 H₂0, 453,0 g Kupfernitrat · 3 H₂0 und 406,0 g Lanthannitrat · 6 H₂0 in 1,2 l Wasser gelöst. Die Umsetzung wurde wie eingangs beschrieben durchgeführt.

### Beispiel 2: (Cr/Cu/Si/Ba/Mn/Ce)

In Rührbehälter 1 wurden 187,5 g Chromoxid, gelöst in 1,2 l Wasser, mit 1,8 l 25%igem Ammoniak und 62,8 g Ludox AS 40 unter Rühren versetzt. In Rührbehälter 2 wurden 11,0 g Bariumnitrat, 38,2 g Mangannitrat · 4 H₂0, 453,0 g Kupfernitrat · 3 H₂0 und 217,1 g Cernitrat · 6 H₂0 in 1,2 l Wasser gelöst. Die Umsetzung wurde wie eingangs beschrieben durchgeführt.

### Beispiel 3: (Cr/Cu/Si/Ba/Mn/Mg)

In Rührbehälter 1 wurden 2048 g Chromoxid, gelöst in 11,25 l Wasser, mit 1,8 l 25%igem Ammoniak und 687 g Ludox AS 40 unter Rühren versetzt. In Rührbehälter 2 wurden 106,0 g Bariumnitrat, 368,0 g Mangannitrat · 4 H₂0 und 4366 g Kupfernitrat · 3 H₂0 in 5 l Wasser gelöst und mit einer Lösung von 1340,0 g Magnesiumnitrat in 6,25 l Wasser versetzt. Die Umsetzung wurde wie eingangs beschrieben durchgeführt.

### Beispiel 4: (Cr/Cu/Si/Ba/Mn/Ag)

In Rührbehälter 1 wurden 187,5 g Chromoxid, gelöst in 1,2 l Wasser, mit 1,8 l 25%igem Ammoniak und 62,8 g Ludox AS 40 unter Rühren versetzt. In Rührbehälter 2 wurden 11,0 g Bariumnitrat, 38,2 g Mangannitrat · 4 H₂0, 453,0 g Kupfernitrat · 3 H₂0 und 85,0 g Silbernitrat in 1,2 l Wasser gelöst. Die Umsetzung wurde wie eingangs beschrieben durchgeführt.

### Beispiel 5: (Cr/Cu/Si/Ba/Mn/Ni)

In Rührbehälter 1 wurden 2048,0 g Chromoxid, gelöst in 11,25 l Wasser, mit 1,8 l 25%igem Ammoniak und 687,0 g Ludox AS 40 unter Rühren versetzt. In Rührbehälter 2 wurden 106,0 g Bariumnitrat, 368,0 g Mangannitrat · 4 H₂0 und 2627,0 g Nickelnitrat · 3 H₂O in 5 l Wasser gelöst und mit einer Lösung von 4366,0 g Kupfernitrat in 6,25 l Wasser versetzt. Die Umsetzung wurde wie eingangs beschrieben durchgeführt.

### Beispiel 6: (Cr/Cu/Si/Ba/Mn/Fe)

In Rührbehälter 1 wurden 2048 g Chromoxid, gelöst in 11,25 l Wasser, mit 1,8 l 25%igem Ammoniak und 687,0 g Ludox AS 40 unter Rühren versetzt. In Rührbehälter 2 wurden 106,0 g Bariumnitrat, 368,0 g Mangannitrat · 4 H₂0 und 3648,0 g Eisennitrat · 9 H₂O in 5 l Wasser gelöst und mit einer Lösung von 4366,0 g Kupfernitrat· 3 H₂0 in 6,25 l Wasser versetzt. Die Umsetzung wurde wie eingangs beschrieben durchgeführt.

### Beispiel 7: (Cr/Cu/Si/Ba/Mn/Ca)

In Rührbehälter 1 wurden 2048 g Chromoxid, gelöst in 11,25 l Wasser, mit 1,8 l 25%igem Ammoniak und 687 g Ludox AS 40 unter Rühren versetzt. In Rührbehälter 2 wurden 106,0 g Bariumnitrat, 368,0 g Mangannitrat · 4 H₂0 und 2133,0 g Calciumnitrat · 4 H₂0 in 5 l Wasser gelöst und mit einer Lösung von 4366 g Kupfernitrat · 3 H₂0 in 6,25 l Wasser versetzt. Die Umsetzung wurde wie eingangs beschrieben durchgeführt.

### Beispiel 8: (Cr/Cu/Si/Ba/Mn/Ag)

In Rührbehälter 1 wurden 187,5 g Chromoxid, gelöst in 1,2 l Wasser, mit 1,8 l 25 %igem Ammoniak und 62,8 g Ludox AS 40 unter Rühren versetzt. In Rührbehälter 2 wurden 11,0 g Bariumnitrat, 38,2 g Mangannitrat · 4 H₂0, 453,0 g Kupfernitrat · 3 H₂0 und 159,4 g Silbernitrat in 1,2 l Wasser gelöst. Die Umsetzung wurde wie eingangs beschrieben durchgeführt.

### Beispiel 9: (Cr/Cu/Si/Ba/Mn/Ce)

In Rührbehälter 1 wurden 187,5 g Chromoxid, gelöst in 1,2 l Wasser, mit 1,8 l 25 %igem Ammoniak und 62,8 g Ludox AS 40 unter Rühren versetzt. In Rührbehälter 2 wurden 11,0 g Bariumnitrat, 38,2 g Mangannitrat · 4 H₂0, 453,0 g Kupfernitrat · 3 H₂0 und 407,3 g Cernitrat · 6 H₂0 in 1,2 l Wasser gelöst. Die Umsetzung wurde wie eingangs beschrieben durchgeführt.

### Beispiel 10: (Cr/Cu/Si/Ba/Mn/Al)

In Rührbehälter 1 wurden 187,5 g Chromoxid, gelöst in 1,2 l Wasser, mit 1,8 l 25%igem Ammoniak und 62,8 g Ludox AS 40 unter Rühren versetzt. In Rührbehälter 2 wurden 11,0 g Bariumnitrat, 38,2 g Mangannitrat · 4 H₂0, 453,0 g Kupfernitrat · 3 H₂0 und 407,3 g Aluminiumnitrat · 9 H₂0 in 1,2 l Wasser gelöst. Die Umsetzung wurde wie eingangs beschrieben durchgeführt.

### Beispiel 11: (Cr/Cu/Si/Ba/Mn/Ca)

In Rührbehälter 1 wurden 187,5 g Chromoxid, gelöst in 1,2 l Wasser, mit 1,8 l 25%igem Ammoniak und 62,8 g Ludox AS 40 unter Rühren versetzt. In Rührbehälter 2 wurden 11,0 g Bariumnitrat, 38,2 g Mangannitrat · 4 H₂0, 453,0 g Kupfernitrat · 3 H₂0 und 255,3 g Calciumnitrat · 4 H₂0 in 1,2 l Wasser gelöst. Die Umsetzung wurde wie eingangs beschrieben durchgeführt.

### Beispiel 12: (Cr/Cu/Si/Ba/Mn/Al)

In Rührbehälter 1 wurden 187,5 g Chromoxid, gelöst in 1,2 l Wasser, mit 1,8 l 25%igem Ammoniak und 62,8 g Ludox AS 40 unter Rühren versetzt. In Rührbehälter 2 wurden 11,0 g Bariumnitrat, 38,2 g Mangannitrat · 4 H₂0, 453,0 g Kupfernitrat · 3 H₂0 und 189,9 g Aluminiumnitrat · 9 H₂0 in 1,2 l Wasser gelöst. Die Umsetzung wurde wie eingangs beschrieben durchgeführt.

### Beispiel 13:

Die Calcinierung der Katalysatoren erfolgte entweder satzweise im Kammerofen bei 750 °C innerhalb 12 Stunden oder kontinuierlich im Drehrohr bei einer Temperatur von 900 °C und einer Verweilzeit von 8 Minuten. Die Calcinierungsbedingungen der einzelnen Beispiele sind in Tabelle 1 zusammengefaßt. Im Rührautoklaven wurden 10 g Pulver der nach Beispiel 1 bis 12 hergestellten Katalysatoren bei einer Temperatur von 220°C und einem Wasserstoffdruck von 250 bar mit 500 g Laurinsäuremethylester zur Reaktion gebracht. Die Hydrierumsätze und die Restverseifungszahl sind in Tabelle 1 zusammengefaßt.

### Beispiel 14

Die Hydrierkatalysatoren mit den Fremdmetallen Aluminium und Mangan wurden nach der Calcinierung mit 2 % Polyvinylacetat und 2 % Graphit vermischt, granuliert und zu 4 ^{x} 4 mm Tabletten verpreßt. Die Katalysatortabletten im Schüttungsvolumen von 600 ml wurden mit einem Wasserstoff/Stickstoff-Gemisch (H₂:N₂=1:10) bei 200°C reduziert. Im 1 l Hochdruckrohrreaktor wurden bei einem Wasserstoffdruck von 250 bar und einer Temperatur von 220 bis 275°C pro Stunde 350 bis 500 ml einer C₁₂-Fettsäure über den Katalysator gepumpt. Die Abläufe waren wasserklar. Es wurden folgende Kennzahlen ermittelt:

| | |
|---|---|
| Säurezahl | 0,04 |
| Verseifungszahl | < 2 |
| Hydroxylzahl | 285 bis 293 |

**Tab.1**

| Hydrierkatalysatoren und Hydrierversuche | | | | |
|---|---|---|---|---|
| Bsp. | BET m²/g | TC °C | Rest-VZ | Umsatz % |
| 1 | 10 | 750 | 209 | 16 |
| 2 | 16 | 750 | 199 | 20 |
| 3 | 14 | 750 | 182 | 27 |
| 4 | 25 | 750 | 142 | 40 |
| 5 | 12 | 750 | 142 | 40 |
| 6 | 15 | 750 | 173 | 31 |
| 7 | 10 | 750 | 187 | 25 |
| 8 | 10 | 900 | 190 | 24 |
| 9 | 19 | 900 | 198 | 21 |
| 10 | 42 | 900 | 187 | 25 |
| 11 | 14 | 900 | 191 | 24 |
| 12 | 18 | 900 | 165 | 34 |
| Legende: BET = Innere Katalysatoroberfläche gemäß BET TC = Calciniertemperatur VZ = Restverseifungszahl | | | | |

| Hydrierbedingungen: | |
|---|---|
| Druck | 250 bar H₂; |
| Temperatur | 220°C; |
| Substrat | Laurinsäuremethylester |
| Einwaage | 500 g; |
| Katalysatoreinwaage | 2 Gew.-% bezogen auf den Ester |

## Patentansprüche

1. Verfahren zur Herstellung von säurefesten, zur direkten Hydrierung von Carbonsäuren zu Alkoholen geeigneten Cupfer-Chrom-Katalysatoren, **dadurch gekernzeichnet**, daß man
a) wäßrige Lösungen, enthaltend (a1) Chrom(VI)oxid und (a2) alkalisch stabilisierte kolloidale Kieselsäure in Mengen von 2 bis 15 Gew.-% - berechnet als Feststoff an Siliciumdioxid und bezogen auf den Feststoffgehalt der Lösung - mit im wäßrigen Milieu alkalisch reagierenden Verbindungen versetzt,
b) die gebildeten Suspensionen mit wäßrigen Lösungen, enthaltend Salze des (b1) Cupfers, (b2) Bariums in Mengen von 0,2 bis 0,9 Gew.-% - berechnet als Metall und bezogen auf den fertigen Katalysator - und (b3) Mangans in Mengen von 3 bis 6 Gew.-% - berechnet als Metall und bezogen auf den fertigen Katalysator - sowie weitere Salze von Metallen ausgewählt aus der Gruppe, die von Calcium, Magnesium, Aluminium, Nickel, Eisen, Cer, Lanthan und Silber gebildet wird, in Mengen von 30 bis 100 Mol-% - berechnet als Metall und bezogen auf den Cupfer-(II)-oxid-Anteil im fertigen Katalysator - behandelt,
c) die Reaktionsmischungen bei erhöhter Temperatur zusammenmischt sowie
d) die gebildeten Feststoffe abtrennt, trocknet, bei einer Temperatur von 750 bis 1050°C calciniert, gegebenenfalls granuliert und anschließend in stückige Form bringt.

2. Verfahren nach Anspruch 1, **dadurch gekennzeichnet**, daß man als alkalisch reagierende Verbindungen Basen einsetzt, die ausgewählt sind aus der Gruppe, die von Ammoniak, Alkali- und Erdalkalihydroxiden und alkalisch reagierenden Aminen gebildet wird.

3. Verfahren nach den Ansprüchen 1 und 2, **dadurch gekennzeichnet**, daß man als Metallsalze Metallnitrate einsetzt.

4. Verfahren nach den Ansprüchen 1 bis 3, **dadurch gekennzeichnet**, daß man die Katalysatoren nach Calcinierung und gegebenenfalls Granulierung mit Bindemitteln und/oder Gleitmitteln versetzt und anschließend in stückige Form bringt.

5. Verwendung von säurefesten Cupfer-Chrom-Katalysatoren nach dem Verfahren nach den Ansprüchen 1 bis 4 zur Direkthydrierung von Fettsäuren zu Fettalkoholen.

6. Verwendung nach Anspruch 5, **dadurch gekennzeichnet**, daß man gesättigte und/oder ungesättigte Fettsäuren und/oder Fettsäuregemische mit 6 bis 24 Kohlenstoffatomen einsetzt.

## Claims

1. A process for the production of acid-resistant copper-chromium catalysts suitable for the direct hydrogenation of carboxylic acids to alcohols, characterized in that
a) compounds showing an alkaline reaction in aqueous medium are added to aqueous solutions containing (a1) chromium(VI) oxide and (a2) alkali-stabilized colloidal silica in quantities of 2 to 15% by weight, expressed as silicon dioxide solids and based on the solids content of the solution,
b) the suspension formed is treated with aqueous solutions containing salts of (b1) copper, (b2) barium in quantities of 0.2 to 0.9% by weight, expressed as metal and based on the final catalyst, and (b3) manganese in quantities of 3 to 6% by weight, expressed as metal and based on the final catalyst, and other salts of metals selected from the group consisting of calcium, magnesium, aluminium, nickel, iron, cerium, lanthanum and silver in quantities of 30 to 100 mol-%, expressed as metal and based on the percentage copper(II) oxide content of the final catalyst,
c) the reaction mixtures are mixed together at elevated temperature and
d) the solids formed are separated off, dried, calcined at a temperature of 750 to 1050°C, optionally granulated and then converted into particulate form.

2. A process as claimed in claim 1, characterized in that bases selected from the group consisting of ammonia, alkali metal and alkaline earth metal hydroxides and alkaline amines are used as the compounds showing an alkaline reaction.

3. A process as claimed in claims 1 and 2, characterized in that metal nitrates are used as the metal salts.

4. A process as claimed in claims 1 to 3, characterized in that binders or lubricants are added to the catalysts after calcination and optionally granulation, after which the catalysts are converted into particulate form.

5. The use of acid-resistant copper-chromium catalysts produced by the process claimed in claims 1 to 4 for the direct hydrogenation of fatty acids to fatty alcohols.

6. The use claimed in claim 5, characterized in that saturated and/or unsaturated fatty acids and/or fatty acid mixtures containing 6 to 24 carbon atoms are used.

## Revendications

1. Procédé d'obtention de catalyseurs cuivre/chrome appropriés pour l'hydrogénation directe d'acides carboxyliques en alcools, résistant aux acide, caractérisé en ce que l'on :
a) mélange des solutions aqueuses contenant : (a1) de l'oxyde de chrome (VI) et, (a2) de l'acide silicique colloïdal stabilisé en milieu alcalin, en quantités allant de 2 à 15 % en poids, calculées comme matière solide en dioxyde de silicium et rapportées à la teneur en solide de la solution, avec des composés à réaction alcaline en milieu aqueux,
b) traite les suspensions formées avec des solutions aqueuses contenant des sels de (b1) cuivre, (b2) de baryum en quantités allant de 0,2 à 0,9 % en poids, calculées comme métal et rapportées au catalyseur terminé et, (b3) de manganèse en quantités allant de 3 à 6 % en poids, calculées comme métal et rapportées au catalyseur terminé, ainsi que d'autres sels de métaux choisis dans le groupe qui est formé par le calcium, le magnésium, l'aluminium, le nickel, le fer, le cérium, le lanthane et l'argent, en quantités allant de 30 à 100 % molaire, calculées comme métal et rapportées au pourcentage d'oxyde de cuivre (II) dans le catalyseur terminé,
c) mélange ensemble les mélanges réactionnels à température élevée et
d) sépare les solides formés, sèche, calcine à une température de 750 à 1050°C, granule le cas échéant et ensuite on les amène sous forme de fragments.

2. Procédé selon la revendication 1, caractérisé en ce que l'on met en oeuvre comme substances à réaction alcaline, des bases qui sont choisies dans le groupe qui est formé de l'ammoniac des hydroxydes de métal alcalin et de métal alcalino-terreux, et des amines à réaction alcaline.

3. Procédé selon les revendications 1 et 2, caractérisé en ce que l'on met en oeuvre comme sels métalliques, des nitrates de métaux.

4. Procédé selon les revendications 1 à 3, caractérisé en ce que l'on additionne les catalyseurs après calcination et le cas échéant granulation, d'agents liants et/ou d'agents de glissement et ensuite on amène sous forme de fragments.

5. Utilisation des catalyseurs cuivre/chrome résistant aux acides, préparés selon le procédé correspondant aux revendications 1 à 4, en vue de l'hydrogénation directe des acides gras en alcools gras.

6. Utilisation selon la revendication 5, caractérisée en ce que l'on met en oeuvre des acides gras saturés et/ou non saturés et/ou des mélanges d'acides gras ayant de 6 à 24 atomes de carbone.
